# FASCICULE DE BREVET EUROPEEN

(11) **EP 0 786 442 B1**
(45) Date de publication et mention de la délivrance du brevet: **26.04.2000**
(21) Numéro de dépôt: 97400148.9
(22) Date de dépôt: 23.01.1997
(51) Int. Cl.: C07C 17/16, C07C 19/01

(54) **Procédé de préparation de chlorures d'alkyle**
Verfahren zur Herstellung von Alkylchloriden
Process for the preparation of alkylchlorides

(30) Priorité: 24.01.1996 FR 9600777
(43) Date de publication de la demande: 30.07.1997
(73) Titulaire: SOCIETE NATIONALE DES POUDRES ET EXPLOSIFS, F-75181 Paris Cédex 04 (FR)
(72) Inventeur: Demail, Hervé, 91710 Vert Le Petit (FR); Le Gars, Pierre, 84700 Sorgues (FR); Schweickert, Jean-Claude, 91710 Vert Le Petit (FR)

(56) Documents cités:
- DE-C- 857 350
- DATABASE WPI Section Ch, Week 7339 Derwent Publications Ltd., London, GB; Class E16, AN 73-58021 XP002015554 & JP 48 030 606 A (HOKKO CHEMICAL IND CO LTD)
- CHEMICAL ABSTRACTS, vol. 100, no. 1, 2 Janvier 1984 Columbus, Ohio, US; abstract no. 5828u, MITSUI TOATSU CHEMICALS, INC.: "Alkyl chlorides" page 500; colonne 1; XP002015553 & JP 58 144 328 A (MITSUI TOATSU CHEMICALS,INC.) 27 Août 1983

## Description

L'invention concerne un procédé amélioré de préparation de chlorures d'alkyle à partir des alcools primaires correspondants et d'acide chlorhydrique.

Il est connu de préparer des chlorures d'alkyle à partir des alcools correspondants en faisant réagir de l'acide chlorhydrique gazeux sur l'alcool en présence de catalyseurs tels que le chlorure de zinc, des amines, des halogénures d'ammonium ou de phosphonium. Cependant ces procédés ne donnent pas entière satisfaction. La durée de réaction est souvent très longue. Des composés isomères se forment. Afin d'augmenter le rendement et de diminuer le temps de réaction, on utilise une très grande quantité de catalyseurs, par exemple environ 27% en masse comme indiqué dans le Chemical Abstracts CA 83 : 27529. Cependant la transformation de l'alcool n'est jamais totale et la séparation de l'alcool et du chlorure d'alkyle est, soit impossible, soit extrêmement difficile en raison des points d'ébullition très proches des composés. La distillation doit être effectuée avec des colonnes possédant de très nombreux plateaux. L'obtention de chlorures d'alkyle avec une très grande pureté est par conséquent ou impossible ou très longue et très coûteuse.

Il existait par conséquent un besoin en un procédé de préparation des chlorures d'alkyle qui ne présente pas les inconvénients des procédés antérieurs.

L'invention a pour objet un procédé de préparation des chlorures d'alkyle caractérisé en ce que :
a) dans une première étape, on fait réagir un alcool aliphatique mono- ou polyhydrique primaire, ayant une chaîne carbonée en C₄ à C₃₀, saturée, substituée ou non par un ou plusieurs atomes de chlore, ramifiée ou non et pouvant comporter un ou plusieurs groupes cycloalkyle en C₅ à C₇, avec de l'acide chlorhydrique gazeux, en absence de catalyseur et à une température comprise entre 80°C et 170°C, jusqu'à ce que le taux de transformation des fonctions alcool soit égal ou compris entre 60% et 95% en mole, et
b) dans une deuxième étape, on introduit dans le milieu réactionnel du phosgène et un catalyseur choisi dans le groupe constitué par les halogénures d'hexaalkylguanidinium et leurs halogénohydrates, les halogénures d'ammonium quaternaires, les halogénures de phosphonium quaternaires, la pyridine, et les pyridines substituées par un ou plusieurs groupes alkyle en C₁ à C₆, à une température comprise entre 80°C et 160°C.

Le procédé selon l'invention permet de transformer quasiment complètement la ou les fonctions alcool en fonction chlorure. Les chlorures peuvent être alors facilement séparés des produits secondaires éventuellement formés, par exemple par distillation. Ils sont par conséquent obtenus avec une très grande pureté, pour certains supérieure à 98,5%.

Les alcools qui sont intéressants à transformer par le procédé selon l'invention sont les alcools aliphatiques mono- ou polyhydriques primaires, en particulier mono-, di- ou trihydriques, saturés, en C₄ à C₃₀, de préférence en C₄ à C₂₂, dont le squelette de la chaîne est uniquement constitué par des atomes de carbone. Cette chaîne peut être linéaire ou ramifiée. Elle peut également comporter un ou plusieurs groupes cycloalkyle tels que les groupes cyclopentyle, cyclohexyle et cycloheptyle. Elle peut porter comme substituants un ou plusieurs atomes de chlore.

Comme exemple d'alcools, on peut citer le butanol-1, le butanediol-1,4, l'hexanol-1, l'hexanediol-1,6, l'octanol-1, le chloro-8 octanol-1, l'octanediol-1,8, le cyclohexylméthanol, le dodécanol-1, l'hexadécanol-1, le docosanol, l'éthyl-2 hexanol-1.

Le procédé est de préférence mis en oeuvre sans solvant. Lorsqu'un solvant est utilisé, il est choisi parmi les solvants inertes vis à vis des réactifs, tels que les solvants aromatiques chlorés.

Dans la première étape, l'alcool est mis à réagir avec de l'acide chlorhydrique gazeux, de préférence sous une pression supérieure à la pression atmosphérique, pouvant aller jusqu'à une pression absolue de 10 bars et en particulier de 2 à 6 bars absolus.

La température de la réaction est comprise entre 80°C et 170°C et de préférence entre 100°C et 150°C.

Aucun catalyseur n'est ajouté dans le milieu réactionnel. On évite ainsi la formation de composés isomères.

La durée de la réaction dépend de l'alcool à transformer. Elle est généralement de plusieurs heures. Lorsque un taux de transformation des fonctions alcool égal ou compris entre 60% et 95% en mole, de préférence égal ou compris entre 70% et 90%, est atteint, on arrête la réaction.

Une phase aqueuse s'est formée. Le chlorure obtenu, l'alcool non transformé et éventuellement des produits secondaires sont contenus dans la phase organique.

Dans la deuxième étape, on introduit du phosgène et un catalyseur dans le milieu réactionnel, de préférence après avoir éliminé la phase aqueuse par exemple après décantation.

Comme catalyseurs, on peut utiliser des halogénures d'ammonium ou de phosphonium quaternaires. L'halogène est le chlore ou le brome et de préférence le chlore. Comme exemple de catalyseurs de ce type, on peut citer le chlorure de triméthylbenzylammonium, le chlorure de tributylbenzylammonium, le chlorure de tétrabutylammonium, le chlorure de tétrahexylammonium, le chlorure de benzyltriphénylphosphonium, le bromure de tétrabutylammonium.

On peut également utiliser pour catalyser la réaction la pyridine ou une pyridine substituée par un ou plusieurs groupes alkyle en C₁ à C₆ telle que la picoline. Dans cette famille, on utilise de préférence la pyridine.

Les catalyseurs préférés sont les halogénures d'hexaalkylguanidinium et leurs halogénohydrates. L'halogène peut être le chlore ou le brome. De préférence, on choisit le chlore. Les radicaux alkyles, identiques ou différents, contiennent généralement de 1 à 8 et de préférence de 1 à 4 atomes de carbone. A titre d'exemples, on peut citer le chlorure d'hexaméthyl-guanidinium, le chlorure d'hexaéthylguanidinium, le chlorure d'hexabutylguanidinium et leurs chlorhydrates. Le chlorure d'hexabutylguanidinium ou son chlorhydrate convient bien.

Généralement, on utilise une quantité de 0,01% à 1% en mole, de préférence de 0,05% à 0,5%, de catalyseur par rapport à la quantité molaire de groupes hydroxyle restant à transformer.

Préférentiellement, on ajoute d'abord le catalyseur dans le milieu réactionnel puis progressivement le phosgène.

La quantité de phosgène introduite est généralement de 1 à 3 moles et de préférence de 1 à 1,5 mole par mole de groupes hydroxyle restant à transformer.

La réaction est effectuée à une température de 80°C à 160°C et de préférence à une température de 100°C à 150°C. Elle dure habituellement de 2 à 15 heures. La phase organique, à la fin de la réaction, ne contient alors pratiquement plus d'alcool.

Après avoir éliminé les gaz restants, on peut si nécessaire, séparer les chlorures des produits secondaires formés tels que les éthers, par exemple par distillation avec une colonne à quelques plateaux, leurs points d'ébullition étant très différents.

On obtient, par le procédé de l'invention, les chlorures avec un bon rendement et une pureté très élevée, souvent supérieure à 98,5%.

Les chlorures d'alkyle sont des composés connus qui sont très utiles en tant que tels ou comme intermédiaires de synthèse, notamment pour la stabilisation des polymères et dans le domaine de la protection des plantes.

Les exemples qui suivent illustrent l'invention sans toutefois la limiter.

### Exemple 1 : Préparation du chlorure d'octyle.

Dans un réacteur de 40 1, sous agitation, on introduit 26,64 kg (205 mol) d'octanol-1, on le chauffe à 125°C et on ajoute, au moyen d'une canne plongeante en tantale, 7,1 kg (194,5 mol) d'acide chlorhydrique gazeux, pendant 8 heures, sous une pression absolue comprise entre 3,9 et 4,5 bars, en maintenant la température entre 125°C et 140°C. On refroidit le mélange jusqu'à 80°C. On décomprime l'acide chlorhydrique gazeux résiduel. On sépare par décantation la phase organique et la phase aqueuse. On récupère alors 28,96 kg de phase organique dont la composition massique déterminée par analyse chromatographique en phase gazeuse (CPG) est la suivante : chlorure d'octyle 78,5%, octanol 16,5%, oxyde de dioctyle 5%.

On introduit dans un réacteur de 25 l, sous agitation, 16,54 kg de la phase organique obtenue précédemment, on ajoute 10 g (0,02 mol) de chlorhydrate de chlorure d'hexabutylguanidinium et on chauffe le mélange à 100°C. On introduit ensuite 2,8 kg (28,3 mol) de phosgène au moyen d'une canne plongeante en verre, pendant 1 heure à une température comprise entre 110°C et 115°C puis on maintient le mélange réactionnel pendant 3 heures à 115°C. On élimine par dégazage le phosgène en excès et l'acide chlorhydrique formé. On obtient ainsi 16,6 kg de phase organique dont la composition massique, déterminée par analyse CPG est la suivante : chlorure d'octyle 93%, octanol 0%, oxyde de dioctyle 5%. Par distillation, au moyen d'une colonne à garnissage (anneaux de Raschig) de 2 m, on récupère 14,3 kg de chlorure d'octyle (point d'ébullition : 107-112°C, sous 50 mm Hg, rendement : 82% par rapport à l'octanol introduit au début du procédé) dont la pureté est de 99,7% (déterminée par analyse CPG).

### Exemple 2 : Préparation du chlorure d'hexadécyle.

Dans un réacteur de 40 l, sous agitation, on introduit 50,14 kg (202 mol) d'hexadécanol-1, on le chauffe à 130°C environ et on introduit 5,2 kg d'acide chlorhydrique gazeux en maintenant la température aux environs de 140°C et la pression entre 4 et 4,5 bars. La réaction terminée, on refroidit le mélange à 80-85°C. On décomprime le réacteur, puis on sépare par décantation la phase organique et la phase aqueuse. La composition massique de la phase organique est d'environ 68% de chlorure d'hexadécyle, 28% d'alcool résiduel et 4% d'oxyde de di-hexadécyle (le rapport du chlorure à l'alcool ayant été déterminé par analyse CPG et le taux d'oxyde ayant été estimé par analyse RMN¹H).

On fait ensuite réagir le phosgène dans la phase organique à une température de 120-125°C en présence de 28 g de chlorhydrate de chlorure d'hexabutylguanidinium en utilisant 10,9 kg de phosgène. On maintient le mélange réactionnel à cette température pendant 5 heures. On élimine par dégazage sous vide le phosgène en excès et l'acide chlorhydrique formé. La phase organique obtenue a la composition massique suivante, déterminée par analyse RMN¹H : chlorure d'hexadécyle 89%, carbonate de dihexadécyle 6,7%, oxyde de dihexadécyle 4,2%.

### Exemple 3 : Préparation du chlorure d'éthyl-2 hexyle.

Dans un réacteur de 40 l, sous agitation, on introduit 26 kg (200 mol) d'éthyl-2 hexanol-1, on le chauffe à 110°C environ et on introduit 4,75 kg d'acide chlorhydrique gazeux en maintenant la température aux environs de 140°C et la pression entre 3,8 et 4,8 bars. La réaction terminée, on refroidit le mélange réactionnel à 80-85°C. On décomprime le réacteur, puis on sépare par décantation la phase organique et la phase aqueuse. On récupère 28,28 kg de phase organique dont la composition massique, déterminée par analyse CPG, est de 53,6% de chlorure d'éthyl-2 hexyle, 41,8% d'alcool résiduel et de 2,3% d'oxyde de di-éthyl-2 hexyle.

On phosgène ensuite 584 g de la phase organique obtenue précédemment, à une température de 110-115°C, en présence de 0,1% en mole par rapport à la quantité molaire d'alcool résiduel, de chlorhydrate de chlorure d'hexabutylguanidinium en utilisant 205 g de phosgène. On maintient le mélange réactionnel à cette température pendant 3 heures puis on élimine par dégazage sous vide le phosgène en excès et l'acide chlorhydrique formé. La phase organique a la composition massique suivante, déterminée par analyse CPG : chlorure d'éthyl-2 hexyle 74,5%, carbonate de bis(éthyl-2 hexyle) 10,7%, chloroformiate d'éthyl-2 hexyle 9,7%, oxyde de bis(éthyl-2 hexyle) 2,4%. Par distillation, on récupère le chlorure d'éthyl-2 hexyle dont la pureté (déterminée par analyse CPG) est supérieure à 97%.

## Revendications

1. Procédé de préparation de chlorures d'alkyle, caractérisé en ce que :
a) dans une première étape, on fait réagir un alcool aliphatique mono- ou polyhydrique primaire, ayant une chaîne carbonée en C₄ à C₃₀, saturée, substituée ou non par un ou plusieurs atomes de chlore, ramifiée ou non et pouvant comporter un ou plusieurs groupes cycloalkyle en C₅ à C₇, avec de l'acide chlorhydrique gazeux, en absence de catalyseur et à une température comprise entre 80°C et 170°C, jusqu'à ce que le taux de transformation des fonctions alcool soit égal ou compris entre 60% et 95% en mole, et
b) dans une deuxième étape, on introduit dans le milieu réactionnel du phosgène et un catalyseur choisi dans le groupe constitué par les halogénures d'hexaalkylguanidinium et leurs halogénohydrates, les halogénures d'ammonium quaternaires, les halogénures de phosphonium quaternaires, la pyridine, et les pyridines substituées par un ou plusieurs groupes alkyle en C₁ à C₆, à une température comprise entre 80°C et 160°C.

2. Procédé selon la revendication 1, caractérisé en ce que la première étape est effectuée sous pression supérieure à la pression atmosphérique.

3. Procédé selon la revendication 1, caractérisé en ce que la quantité de catalyseur utilisée est de 0,01% à 1% en mole par rapport à la quantité molaire de groupes hydroxyle restant à transformer.

4. Procédé selon la revendication 1, caractérisé en ce que le catalyseur est ajouté avant l'introduction du phosgène.

5. Procédé selon la revendication 1, caractérisé en ce que le phosgène est ajouté progressivement.

6. Procédé selon la revendication 1, caractérisé en ce qu'on élimine la phase aqueuse formée au cours de la première étape avant d'effectuer la deuxième étape.

7. Procédé selon la revendication 1, caractérisé en ce que la quantité de phosgène introduite est de 1 à 3 mole par mole de groupes hydroxyle à transformer.

8. Procédé selon la revendication 1, caractérisé en ce que le catalyseur est choisi dans le groupe constitué par les halogénures d'hexaalkylguanidinium et leurs halogénohydrates.

9. Procédé selon la revendication 8, caractérisé en ce que le catalyseur est le chlorure d'hexabutylguanidinium ou son chlorhydrate.

## Patentansprüche

1. Verfahren zur Herstellung von Alkylchloriden,
dadurch gekennzeichnet, daß:
a) in einem ersten Schritt ein verzweigter oder nicht verzweigter, gegebenenfalls mit einem oder mehreren Chloratomen substituierter, gesättigter, aliphatischer, primärer Alkohol mit einer oder mehreren Hydroxygruppen, der eine Kohlenstoffkette mit 4 bis 30 Kohlenstoffatomen aufweist und eine oder mehrere C₅₋₇-Cycloalkylgruppen aufweisen kann, mit gasförmiger Chlorwasserstoffsäure in Abwesenheit eines Katalysators bei einer Temperatur im Bereich von 80 bis 170 °C umgesetzt wird, bis der Umwandlungsgrad der Alkoholgruppen im Bereich von 60 bis 95 Mol-% liegt, und
b) in einem zweiten Schritt in das Reaktionsmedium bei einer Temperatur im Bereich von 80 bis 160 °C Phosgen und ein Katalysator gegeben wird, der unter den Hexaalkylguanidiniumhalogeniden und deren Hydrohalogeniden, den Halogeniden von quartären Ammoniumverbindungen, den Halogeniden von quartären Phosphoniumverbindungen, Pyridin und den Pyridinen, die mit einer oder mehreren C₁₋₆-Alkylgruppen substituiert sind, ausgewählt ist.

2. Verfahren nach Anspruch 1, dadurch gekennzeichnet, daß der erste Schritt bei einem Druck durchgeführt wird, der über Atmosphärendruck liegt.

3. Verfahren nach Anspruch 1, dadurch gekennzeichnet, daß der verwendete Mengenanteil des Katalysators im Bereich von 0,01 bis 1 Mol-%, bezogen auf die molare Menge der noch umzuformenden Hydroxygruppen, liegt.

4. Verfahren nach Anspruch 1, dadurch gekennzeichnet, daß der Katalysator vor dem Phosgen zugegeben wird.

5. Verfahren nach Anspruch 1, dadurch gekennzeichnet, daß das Phosgen allmählich zugegeben wird.

6. Verfahren nach Anspruch 1, dadurch gekennzeichnet, daß die während des ersten Schritts gebildete wäßrige Phase entfernt wird, bevor der zweite Schritt durchgeführt wird.

7. Verfahren nach Anspruch 1, dadurch gekennzeichnet, daß der Mengenanteil des zugesetzten Phosgens 1 bis 3 mol pro Mol noch umzusetzender Hydroxygruppen beträgt.

8. Verfahren nach Anspruch 1, dadurch gekennzeichnet, daß der Katalysator unter den Hexaalkylguanidiniumhalogeniden und deren Hydrohalogeniden ausgewählt ist.

9. Verfahren nach Anspruch 8, dadurch gekennzeichnet, daß der Katalysator das Hexabutylguanidiniumchlorid oder das Hydrochlorid dieser Verbindung ist.

## Claims

1. Method for preparing alkyl chlorides, characterized in that :
a) in a first step, a primary mono- or polyhydric aliphatic alcohol, having a saturated carbon chain of C₄ to C₃₀, substituted or not with one or more chlorine atoms, which is branched or unbranched and which may include one or more cycloalkyl groups with C₅ to C₇, is reacted with gaseous hydrochloric acid, in the absence of a catalyst and at a temperature of between 80°C and 170°C, until the degree of conversion of the alcohol functional groups is equal to, or included within 60 % and 95 % in moles, and
b) in a second step, phosgene is introduced into the reaction medium, together with a catalyst selected from the group consisting of hexaalkylguanidinium halides and their hydrohalides, quaternary ammonium halides, quaternary phosphonium halides, pyridine and pyridines substituted with one or more C₁ to C₆ alkyl groups, at a temperature of between 80°C and 160°C.

2. Method according to claim 1, characterized in that the first step is carried out under a pressure greater than atmospheric pressure.

3. Method according to claim 1, characterized in that the quantity of catalyst used is from 0.01 % to 1 % in moles with respect to the molar quantity of the hydroxyl groups still to be converted.

4. Method according to claim 1, characterized in that the catalyst is added before phosgene is introduced.

5. Method according to claim 1, characterized in that phosgene is added progressively.

6. Method according to claim 1, characterized in that the aqueous phase formed during the first step is removed before the second step is carried out.

7. Method according to claim l, characterized in that the quantity of phosgene introduced is from 1 to 3 mole per mole of hydroxy groups to be converted.

8. Method according to claim 1, characterized in that the catalyst is selected from the group consisting of hexaalkylguanidinium halides and their hydrohalides.

9. Method according to claim 8, characterized in that the catalyst is hexabutylguanidinium chloride or its hydrochloride.
